# EUROPEAN PATENT APPLICATION

(11) **EP 3 905 462 A1**
(43) Date of publication of application: **03.11.2021**
(21) Application number: 21171295.5
(22) Date of filing: 29.04.2021
(51) Int. Cl.: H02G 1/12, B22F 3/00, B23K 26/00, G03F 7/00, H05K 3/02

(54) **WIRE HANDLING SYSTEM AND METHOD FOR LASER ABLATION**

(30) Priority: 30.04.2020 US 202063017791 P
(71) Applicant: Heraeus Deutschland GmbH & Co KG, 63450 Hanau (DE)
(72) Inventor: SCHUSTER, Paul, St. Paul, 55127 (US); TILLMAN, Darren, St. Paul, 55127 (US); HARRISON, Anthony, St. Paul, 55127 (US); GEBERT, Jörg-Martin, 63450 Hanau (DE)
(74) Representative: Maiwald Patent- und Rechtsanwaltsgesellschaft mbH

(57) **Abstract**

One aspect is an ablation system (10) with a wire feed (12) configured to feed a wire (11) and a wire take-up (14) configured to take-up the wire (11). A wire handling system (20) is configured to advance the wire (11) and to stop the wire (11) between the wire feed (12) and the wire take-up (14) in a controlled manner. A laser ablation processor (22) is located between the wire feed (12) and the wire take-up (14), the laser ablation processor (22) having at least one laser configured to ablate the wire (11) when the (11) wire is stopped. A clamp system (26) is located between wire feed (12) and the wire take-up (14) and configured to clamp onto the wire (11) when the wire (11) is stopped.

## Description

### BACKGROUND

Many medical device applications require stripping the outer layers of materials, such as polymers or various metals, from small diameter wire. One approach is to use a laser to accomplish stripping of one or more outer layers. Laser stripping is a noncontact process that is very repeatable and can be automated. Lasers can selectively remove layers of insulation or areas of insulation. Compared with chemical-based stripping methods, laser stripping is safer, cleaner, less expensive and more sustainable.

Thin-diameter wire is used in various devices for cardiac rhythm management, neurological stimulation and radio frequency ablation. This wire is typically coated with an insulating polymer that must be removed in sections to expose the underlying metal conductor. The diameter of these wires is decreasing, making other stripping methods impractical. At the same time, stripping requirements are getting more complex. In some cases, insulation must be selectively removed from multiple layers, one layer at a time.

As wire diameters in these applications continue to decrease in size, these delicate wires become challenging to process with accuracy and speed. For these and other reasons, there is a need for the present embodiments.

### BRIEF DESCRIPTION OF THE DRAWINGS

Figure 1 illustrates an overview of a wire ablation system in accordance with one embodiment.
Figures 2A-2C illustrate various views of a multi-layer wire for a wire ablation system in accordance with one embodiment.
Figures 3A-3C illustrate sequences of wire handling in a wire ablation system in accordance with one embodiment.
Figures 4A-4B illustrate oscillation reduction for a wire ablation system using a clamp in accordance with one embodiment.
Figure 5 illustrates a method of laser ablation on a wire in accordance with one embodiment.

### DETAILED DESCRIPTION

In the following detailed description, reference is made to the accompanying drawings which form a part hereof, and in which is shown by way of illustration specific examples in which the disclosure may be practiced. It is to be understood that other examples may be utilized and structural or logical changes may be made without departing from the scope of the present disclosure. The following detailed description, therefore, is not to be taken in a limiting sense, and the scope of the present disclosure is defined by the appended claims. It is to be understood that features of the various examples described herein may be combined, in part or whole, with each other, unless specifically noted otherwise.

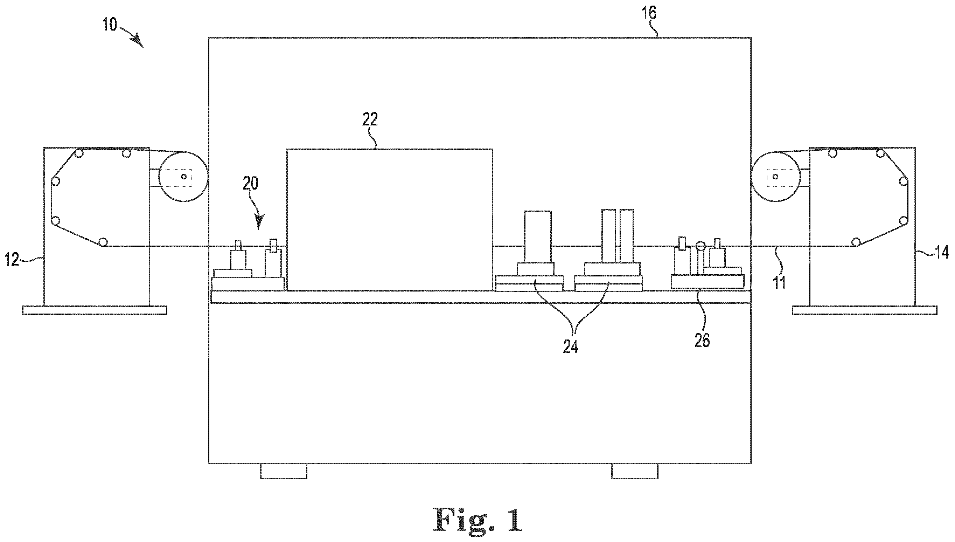

Figure 1 is a wire ablation system 10 in accordance with one embodiment. Wire ablation system 10 includes wire feed 12, wire take-up 14 and wire positioning system 16. In one embodiment, wire positioning system 16 incudes wire handling system 20, laser ablation processor 22, inspection system 24, and wire clamp system 26. In one embodiment, wire ablation system 10 allows for the controlled, accurate and fast processing of a wire 11, such that layers and/or areas of wire 11 can be selectively removed at selected locations along wire 11.

In one embodiment, wire ablation system 10 is a "spool-to-spool" system, wherein a spool of wire 11 is provided at wire feed 12, and an empty spool is provided at wire take-up 14. In one embodiment, a tensioning system is provided at wire feed 12, at wire take-up 14, or at both. Such tensioning systems provide a near constant tension on wire 11, such that wire 11 is tightly held between wire feed 12 and wire take-up 14. The tensioning system in various embodiments include a plurality of rollers and/or weighted rollers and/or springs and/or electromagnetics and/or motors to facilitate the provision of tension on wire 11.

In one embodiment, laser ablation processor 22 includes at least one laser, and is located between wire feed 12 and wire take-up 14. Once wire 11 is in a secure and tightened position extending through laser ablation processor 22, portions of wire 11 can be ablated to remove layers, or portions of layers of wire 11 in a controlled manner at precise locations. After each ablation process within laser ablation processor 22, inspection system 24 can inspect wire 11 as it is advanced to wire take-up 14 to ensure each ablated portion conforms to specifications.

In one embodiment, wire ablation system 10 is used to process wire 11 into medical products, such as sensing and/or stimulation electrode parts for insertion and/or implantation into the human body.

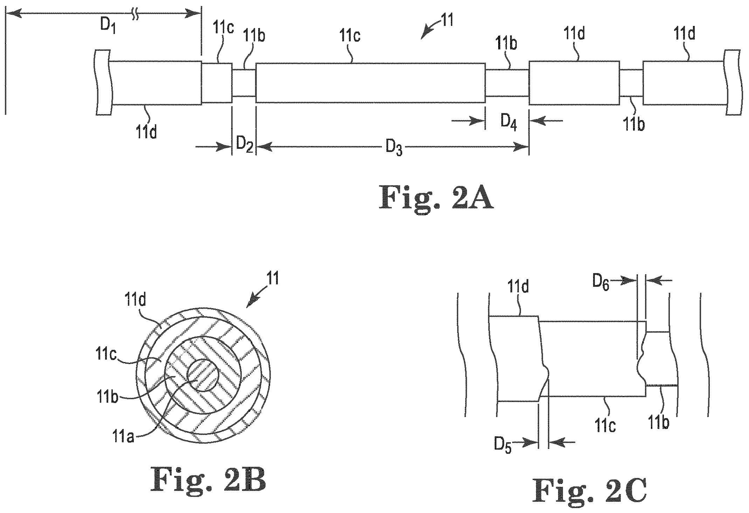

Figures 2A-2C respectively illustrate side, sectional and enlarged views of a multi-layer wire 11 for a wire ablation system 10 in accordance with one embodiment. In one embodiment, wire 11 includes core 11a, first layer 11b, second layer 11c and third layer 11d. In various embodiments, wire ablation system 10 is configured to ablate certain layers of wire 11, or portions of layers. In one embodiment, wire ablation system 10 is configured to ultimately produce wire parts having discrete lengths. In one embodiment, the individual parts are singulated from wire 11 after it is fully collected on wire take-up 14. The distance from the end of one part to the next is illustrated, for example, as a part length D₁ illustrated in Figure 2A. In one example, part length D₁ is 0.955 inches (24.26 mm), with a part tolerance variation of +/- 0.005 inches (0.127 mm). Within each part, any number of ablations can be made to one or more layers along a variety of distances. The number and nature of ablations can be varied from part to part.

In one example illustrated in Figure 2A, second and third layers 11c and 11d are ablated exposing first layer 11b in several locations along the length of wire 11. In one embodiment, the second and third layers 11c and 11d are ablated at one location along a distance D₂, while in another they are ablated along a distance D₄. In one embodiment, distance D₂ is 0.024 inches (0.61 mm), while distance D₄ is 0.050 inches (1.27 mm), with a tolerance variation of +/- 0.001 inches (0.0254 mm). In one embodiment, only third layer 11d is ablated at one location between distance D₂ and distance D₄, exposing second layer 11c. In one embodiment, the distance D₃ between and edge of one ablated section and the next is 0.290 inches (7.366 mm), with a tolerance variation of +/- 0.003 inches (0.0762 mm). As will be apparent to one skilled in the art, any number of various patterns of removing layers along the axial length of wire 11 can be readily achieved.

In one embodiment, multiple lasers are used to remove entire second and third layers 11c and 11d around the full circumference of wire 11 at the specified locations. For example, four laser located at 90 degrees relative to each other around 360 degree circumference of wire 11 can effectively remove an entire layer around the full circumference of wire 11 at the specified locations. In various embodiments, the layers of wire 11 may be conductive material, such as metals and alloys, and may include insulative materials, such as polymers.

Furthermore, very fine tolerances of ablated regions can be achieved in very small diameter wire. For example, as illustrated in Figure 2B, in one embodiment, the respective outer diameters of wire 11 are 0.007 inches (0.1778mm) for third layer 11d, 0.0055 inches (0.1397mm) for second layer 11c, 0.0035 inches (0.0889mm) for first layer 11b and 0.0015 inches (0.0381mm) for core 11a. In other embodiments, other similar diameters are possible. For example, core 11a may have an outer diameter between 0.0010 inches (0.0254mm) and 0.0018 inches (0.04572mm), first layer 11b may have an outer diameter between 0.0030 inches (0.0762mm) and 0.0045 inches (0.1143mm), second layer 11c may have an outer diameter between 0.0045 inches (0.1143mm) and 0.0070 inches (0.1778mm), and third layer 11d may have an outer diameter between 0.0050 inches (0.127mm) and 0.0082 inches (0.2083mm). Other similar small diameters outside these ranges are also possible in accordance with other embodiments.

In one embodiment, the ablation of layers from wire 11 does not make a perfectly perpendicular surface relative to the longitudinal axis of wire 11. As illustrated in Figure 2C, when third layer 11d is ablated exposing second layer 11c, and likewise, when second layer 11 c is ablated exposing first layer 11b, the respective edges of third layer 11d and second layer 11c are not entirely vertical relative to the wire axis. In one embodiment, the unevenness of the edges is contained within tolerance distances D₅ and D₆, which is in one embodiment less than 0.002 inches (0.0508 mm).

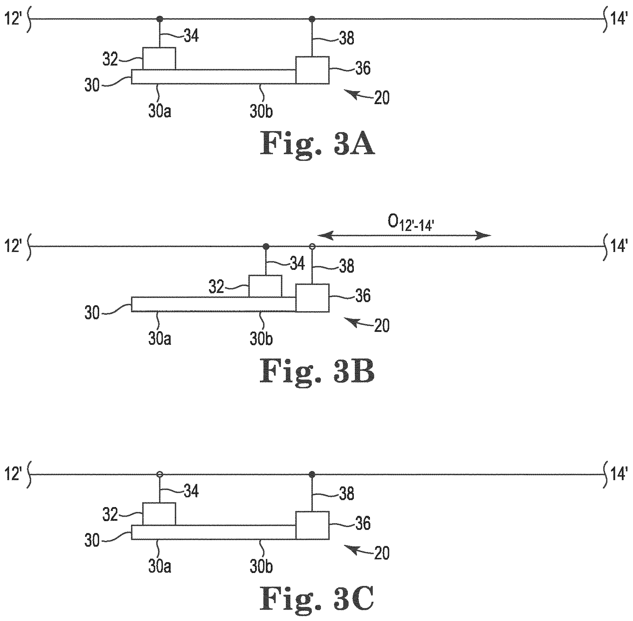

In one embodiment, wire handling system 20 is provided in order to controllably and rapidly move wire 11 through laser ablation processor 22, such that wire 11 can be ablated at various axial locations along its length as described above. In one embodiment, wire handling system 20 is configured for controllably advancing wire 11 from wire feed 12 to wire take-up 14. In one embodiment, wire handling system 20 secures wire 11, advances wire 11, and releases wire 11, in order to prepare for additional advancing, as is further described and illustrated in conjunctions with Figures 3A-3C.

Figures 3A-3C illustrate wire handling system 20 advancing wire 11 in wire ablation system 10 from a wire feed side 12' to a wire take-up side 14' in accordance with one embodiment. In one embodiment, wire handling system 20 includes first portion 30, second portion 32, third portion 34, fourth portion 36 and fifth portion 38. First portion 30 is oriented such that it has a feed side 30a nearer to the wire feed 12 and a take-up side 30b nearer to wire take-up 14. In Figure 3A, second portion 32 is located adjacent feed side 30a of first portion 30, and second portion 32 is movable relative to first portion 30. Third portion 34 is fixed to second portion 32, and in Figure 3A, third portion 34 is coupled to wire 11. Fourth and fifth portions 36 and 38 are fixed relative to first portion 30 and to each other, and in Figure 3A fifth portion 38 is coupled to wire 11.

Figure 3B illustrates wire handling system 28 advancing wire 11 toward wire take-up 14. In one embodiment, second and third portions 32 and 34 are accelerated and moved from feed side 30a to take-up side 30b of first portion 30, thereby advancing wire 11, since third portion 34 is coupled to wire 11. In Figure 3B, fifth portion 38 is released from wire 11 such that it guides wire 11 as it moves from wire feed side 12' to wire take-up side 14'. In one embodiment, the distance that second and third portions 32 and 34 are moved along first portion 30 is D₁, the distance from the end of one part to the next part.

After wire 11 in wire ablation system 10 is accelerated and advanced as illustrated in Figure 3A to that illustrated in Figure 3B, it is then rapidly stopped and secured with fifth portion 38 so that a laser or lasers within laser ablation processor 22 can ablate portions of wire 11. In one embodiment, fifth portion 38 is coupled to wire 11 for the ablation (illustrated in Figure 3C). In one embodiment, multiple locations on wire 11 are ablated, such as illustrated in Figure 2A. While wire 11 is ablated, third portion 34 is released from wire 11 so the second and third portions 32 and 34 can be moved back to the feed side 12' of first portion 30, as illustrated in Figure 3C, in preparation for the next movement of wire 11.

Although Figures 3A-3C illustrated one embodiment for processing and moving wire 11, one skilled in the art understands that there are various similar means of rapidly advancing wire 11 and then quickly stopping wire 11 so that portions can be ablated at set locations.

When wire 11 is configured of very small diameter, however, rapid acceleration followed by abrupt stopping of the advancement of wire 11 also leads to wire oscillation in the direction of the length of the wire. Wire oscillation between wire feed side 12' and wire take-up side 14' is in the direction of the arrow O_{12'-14'} illustrated in Figure 3B. When processing requirements dictate that wire 11 be moved and ablated as quickly as possible, the rapid moving and then stopping of wire 11 only exacerbates wire oscillation O_{12'-14'}. When wire diameter and the distance between ablations and the length of ablations are all very small, as are the tolerance requirements, wire oscillation O_{12'-14'} within laser ablation processor 22 will produce errors in the parts that can lead to part rejection and waste. Furthermore, the time that it takes for wire oscillation O_{12'-14'} to dissipate sufficient to proceed with processing slows down the manufacturing process leading to higher processing costs.

In order to minimize wire oscillation O_{12'-14'} and optimize efficient and accurate wire ablation and processing within laser ablation processor 22, wire clamp system 26 is provided on the wire take-up side 14' of laser ablation processor 22. In one embodiment, as soon as wire handling system 20 has advanced wire 11 to the appropriate location for ablation within laser ablation processor 22, wire clamp system 26 is coupled to wire 11. In this way, each of wire handling system 20 and wire clamp system 26 are coupled to wire 11 prior to and during the ablation process within laser ablation processor 22. It was found that clamping the wire on both sides of laser ablation processor 22 mitigated wire oscillation O_{12'-14'} sufficient to significantly reduce ablation errors and greatly improve tolerances.

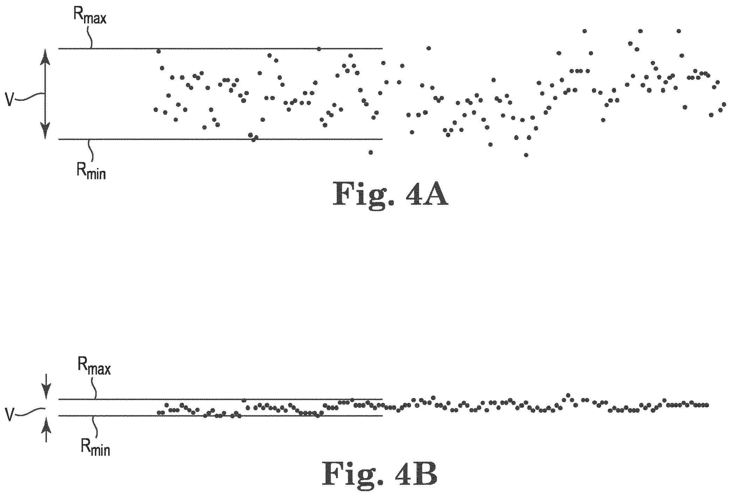

Figures 4A-4B illustrate the improvement in wire ablation system 10, including wire clamp system 26, in accordance with one embodiment. Figure 4A illustrates the variation V in wire positioning where wire 11 is held only on one side of laser ablation processor 22, such as by wire handling system 20. In one case where wire 11 is only held on one side, wire oscillation O_{12'-14'} is significant enough, illustrated in Figure 4A, where wire position variation V between Rₘₐₓ and Rmin is approximately 0.007 inches (0.1778 mm), including measurement error. With wire variation V of up to 0.007 inches (0.1778 mm), this will exceed the required maximum tolerances allowed for many applications, which potentially results in having to waste parts.

Where wire 11 is secured on both sides of laser ablation processor 22, however, such as by wire handling system 20 on the wire feed side 12' of laser ablation processor 22 and by wire clamp system 26 on the wire take-up side 14' of laser ablation processor 22, wire oscillation O_{12'-14'} is substantially reduced. As illustrated in Figure 4B, wire position variation V between Rmax and Rmin is approximately 0.002 inches (0.0508 mm), including measurement error. Because wire 11 is securely clamped on both sides of laser ablation processor 22 just prior to and during the laser ablation of wire 11, wire oscillation O_{12'-14'} is substantially reduced during the ablating, thereby substantially decreasing error in locations of the ablated portions along the wire 11.

Although Figure 1 illustrates wire handling system 20 on the wire feed side 12' of laser ablation processor 22 and wire clamp system 26 on the wire take-up side 14' of laser ablation processor 22, the location of wire handling system 20 and wire clamp system 26 relative to laser ablation processor 22 can also be reversed, such that wire handling system 20 is on the wire take-up side 14' of laser ablation processor 22 and wire clamp system 26 is on the wire feed side 12' of laser ablation processor 22. Having wire handling system 20 and wire clamp system 26 on opposite sides of laser ablation processor 22, however, facilitates securing wire 11 on both sides of laser ablation processor 22 thereby substantially reducing wire oscillation O_{12'-14'} within laser ablation processor 22 during the moving and stopping process.

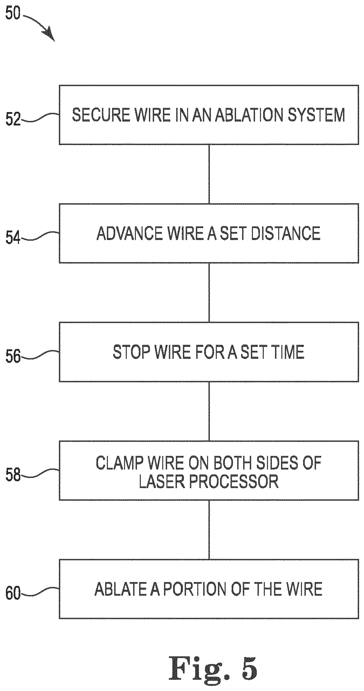

Figure 5 illustrates a method 50 of laser ablation on a wire in accordance with one embodiment. In one embodiment, at 52 a wire is secured within an ablation system, such as wire ablation system 10 described above. In one embodiment, the wire is secured between two spools that are spaced apart such that a portion of the wire extends longitudinally between the spools in such a way that one spool feeds wire to the other spool. In one embodiment, the length of wire that extends between the spools is under tension.

At 54, the wire is advanced within the wire ablation system by a set distance. In one embodiment, the set distance is a part length. For example, the wire may be advanced the length of a medical product part. In other embodiments, differing lengths of advancement can be used.

At 56, the wire is stopped from advancement for a set period of time so that the wire can be processed. At 58, the wire is clamped on both sides of a laser processor after the wire is stopped. In one embodiment, laser processor, such as laser ablation processor 22 described above, is located along a length of the wire between the spools. On each side of the laser processor, the wire is clamped in order to minimize wire oscillation and maximize accuracy and speed of the laser ablation.

Once the wire is clamped, at 60 portions of the wire are ablated in accordance with specifications for the part. As discussed above, within each part, any number of ablations can be made to one or more layers along a variety of distances. The number and nature of ablations can be varied from part to part. Once the ablations are made, the process can be repeated, such as repeating 54-60 as many times as needed. The individual parts can be singulated from the wire take-up as needed.

Although specific examples have been illustrated and described herein, a variety of alternate and/or equivalent implementations may be substituted for the specific examples shown and described without departing from the scope of the present disclosure. This application is intended to cover any adaptations or variations of the specific examples discussed herein. Therefore, it is intended that this disclosure be limited only by the claims and the equivalents thereof.

## Claims

1. An ablation system (10) comprising:
a wire feed (12) configured to feed a wire (11);
a wire take-up (14) configured to take-up the wire (11);
a wire handling system (20) configured to advance the wire (11) and to stop the wire (11) between the wire feed (12) and the wire take-up (14) in a controlled manner;
a laser ablation processor (22) between the wire feed (12) and the wire take-up (14), the laser ablation processor (22) comprising at least one laser configured to ablate the wire (11) when the wire (11) is stopped; and
a clamp system (26) located between the wire feed (12) and the wire take-up (14) and configured to clamp onto the wire (11) when the wire (11) is stopped.

2. The ablation system (10) of a previous claim, wherein the wire handling system (20) and the clamp system (26) are configured to simultaneously clamp the wire while the at least one laser ablates the wire when the wire is stopped.

3. The ablation system (10) of a previous claim, wherein the wire handling system (20) is located on a first side of the laser ablation processor (22) and the clamp system (26) is located on a second side of the laser ablation processor (22), which is opposite the first side.

4. The ablation system (10) of a previous claim, wherein the wire handling system (20) is located between the wire feed (12) and the laser ablation processor (22) and the clamp system (26) is located between the laser ablation processor (22) and the wire take-up (14).

5. The ablation system (10) of a previous claim, wherein the clamp system (26) is located between the wire feed (12) and the laser ablation processor (22) and the wire handling system (20) is located between the laser ablation processor (22) and the wire take-up (14).

6. The ablation system (10) of a previous claim further comprising a wire inspection system located between the laser ablation processor (22) and the wire take-up (14).

7. The ablation system (10) of a previous claim wherein the wire feed (12) and/or the wire take-up (14) further comprises a spool and at least one of wire tensioning rollers, weighted rollers, springs, electromagnetics, and motors.

8. The ablation system (10) of a previous claim wherein the wire is a multilayer medical wire having one, two, three, or four layers comprising a core having an outer diameter between 0.0010 inches (0.0254mm) and 0.0018 inches (0.04572mm), a first layer over the core having an outer diameter between 0.0030 inches (0.0762mm) and 0.0045 inches (0.1143mm), a second layer over the first layer having an outer diameter between 0.0045 inches (0.1143mm) and 0.0070 inches (0.1778mm), and a third layer over the second layer having an outer diameter between 0.0050 inches (0.127mm) and 0.0082 inches (0.2083mm).

9. A method of ablation a wire (11) comprising:
providing a wire (11) on a wire feed (12);
providing a wire take-up (14) configured to take-up the wire;
controlling movement of the wire between the wire feed (12) and the wire take-up spool with a wire handling system (20) configured to advance the wire and to stop the wire in a controlled manner;
laser ablating the wire with at least one laser located between the wire feed (12) and the wire take-up spool when the wire is stopped; and
clamping the wire during the laser ablation at a first location between the wire feed (12) and the at least one laser, and clamping the wire at a second location between the wire take-up and the at least one laser.

10. The method of a previous claim, wherein the wire handling system (20) and a clamp system (26) are configured to simultaneously clamp the wire while the at least one laser ablates the wire when the wire is stopped.

11. The method of a previous claim, wherein the wire handling system (20) is located on a first side of a laser ablation processor (22) and the clamp system (26) is located on a second side of the laser ablation processor (22), which is opposite the first side.

12. The method of a previous claim further comprising inspecting the ablations made on the wire after the laser ablation.

13. The method of a previous claim wherein the wire feed (12) further comprises tensioning the wire between the wire feed and the wire take-up spool using at least one of wire tensioning rollers, weighted rollers, springs, electromagnetics, and motors.

14. The method of a previous claim wherein the wire is a multilayer wire such that at least one portion of at least one layer is ablated during the laser ablating.

15. The method of a previous claim wherein the wire is a multilayer wire such that at least one portion of at least two layers is ablated during the laser ablating.
